# FASCICULE DE BREVET EUROPEEN

(11) **EP 1 885 432 B1**
(45) Date de publication et mention de la délivrance du brevet: **24.12.2008**
(21) Numéro de dépôt: 05762705.1
(22) Date de dépôt: 31.05.2005
(51) Int. Cl.: A61M 35/00, A45D 37/00, B65D 81/22, B65D 81/32

(54) **EMBALLAGE HERMETIQUE POUR SUPPORT IMPREGNE D'UN PRODUIT**
HERMETISCH VERSIEGELTE VERPACKUNG FÜR EINEN MIT EINEM PRODUKT IMPRÄGNIERTEN TRÄGER
HERMETICALLY-SEALED PACKAGE FOR A SUPPORT THAT IS IMPREGNATED WITH A PRODUCT

(43) Date de publication de la demande: 13.02.2008
(73) Titulaire: Laboratoire Tetra Medical, 07100 Annonay (FR)
(72) Inventeur: Dos Santos, Wilton, Adolfo, 67800 Bischheim (FR)
(74) Mandataire: Rhein, Alain
(86) Numéro de dépôt international: PCT/FR2005/050391
(87) Numéro de publication internationale: WO 2006/128980

(56) Documents cités:
- WO-A-03/089330
- US-A- 5 111 934
- US-A1- 2003 004 089
- US-A1- 2003 194 426
- US-B1- 6 326 069

## Description

La présente invention concerne un emballage pour support imprégné d'un produit, support hermétiquement enfermé dans au moins une pochette.

Un tel emballage trouvera son application en pharmacie, cosmétique, en parfumerie ou analogue pour une application cutanée dudit produit.

En général, les pochettes connues, à usage unique, enferment une serviette, comme un rince-doigts, ou analogue. Cette pochette est alors ouverte et la serviette imprégnée d'un produit est extraite de la pochette pour ensuite être apposée sur la peau.

Un inconvénient d'une telle pochette réside tout d'abord dans l'ouverture peu aisée de l'emballage. En effet, ce type de pochette consiste souvent en deux parois scellées ensemble. Chaque paroi se présente sous la forme d'une feuille constituée d'une juxtaposition de plusieurs couches, comme un laminé ou un complexe de laminés de divers polymères. Les couches extérieures protectrices sont réalisées à partir de matériaux plastifiés génère un inconvénient lors de l'ouverture de la pochette qui provoque une déchirure grossière de l'emballage et la formation de bavures. De plus, il arrive parfois que le support soit détérioré lors de l'ouverture de la pochette.

Un autre inconvénient provient de la manipulation dudit support imprégné après ouverture de la pochette de sorte que, d'une part, le produit entre en contact avec les doigts de l'utilisateur et, d'autre part, ledit support peut être souillé lors de sa manipulation. Cet inconvénient pose notamment des problèmes d'hygiène et de contamination qui exclut l'utilisation de ce type de pochette dans le cadre d'un désinfectant ou d'un cicatrisant.

De ce cas précis, il est aussi nécessaire après avoir désinfecté la plaie de la protéger, notamment au moyen d'un pansement, d'un bandage, d'une compresse, d'un sparadrap ou analogue. Mais ces moyens de protection étant distincts du désinfectant, il s'avère qu'on dispose souvent de l'un ou l'autre, mais moins souvent des deux.

Un emballage, décrit dans le document EP 1 296 627, tente de pallier les inconvénients de l'état de la technique en proposant un emballage enfermant dans une pochette un support imprégné de produit pour application cutanée. En particulier, cet emballage est destiné à enfermer un support imprégné d'un produit cicatrisant ou désinfectant. Cette pochette est constituée des deux parois fixées l'une à l'autre par des moyens permettant de désolidariser partiellement lesdites parois par pelage de l'une par rapport à l'autre. Ledit support est fixé par soudure ou collage à au moins une desdites parois de sorte qu'un utilisateur le manipule indirectement au travers de l'emballage. Même s'il apporte entière satisfaction du point de vue de la conservation maximale de la stérilité du support après ouverture, un tel dispositif pose toutefois l'inconvénient d'une manipulation peu aisée en raison notamment de son mode d'ouverture par pelage. Le document US 2003 /0004089 décrit un emballage selon le préambule du révendication 1.

L'invention a pour but de pallier les inconvénients de l'état de la technique en proposant un emballage pour support imprégné de produit dont l'ouverture est facilitée et nette et qui permette de manipuler ledit support sans le salir ou sans qu'il entre directement en contact avec les doigts de l'utilisateur. De plus, selon un mode de réalisation, l'emballage selon l'invention permet de combiner une pochette enfermant un support imprégné avec une autre pochette enfermant un élément pour un usage complémentaire. Par exemple, et de façon non limitative, la première pochette peut renfermer un support imbibé d'un cicatrisant ou d'un désinfectant tandis que l'autre pochette enferme des moyens de protection de la plaie ainsi traitée, comme un pansement ou un sparadrap.

Pour ce faire, l'invention concerne un emballage pour support imprégné, support hermétiquement enfermé dans au moins une pochette, cette dernière comprend des moyens de séparation d'au moins une première partie de la pochette laissant apparaître au moins partiellement ledit support imprégné solidaire d'une deuxième partie de ladite pochette apte à servir de moyen de préhension dudit support imprégné, lesdits moyens de séparation se présentent sous la forme d'au moins une zone d'affaiblissement des matériaux constituant ladite pochette de manière à faciliter la séparation de la première et de la deuxième partie de ladite pochette, au travers d'au moins une ligne de pré-découpe s'étendant au moins en partie transversalement à ladite pochette et comprenant des découpes ou des perforations au moins partielles desdits matériaux constituant ladite pochette de manière à conserver l'étanchéité de ladite pochette, caractérise par le fait que ladite pochette comprend des parois constituées d'une superposition de trois couches et que lesdites découpes ou perforations sont réalisées au travers de deux desdits couches, tout en conservant une des couches intacte.

De plus les découpes ou perforations sont réalisées au travers d'au moins une paroi interne et/ou externe de ladite pochette, tout en conservant une paroi intacte.

Selon un mode de réalisation, la zone d'affaiblissement comprend au moins une ligne de pré-découpe s'étendant au moins en partie transversalement à ladite pochette et au moins une encoche située en périphérie de ladite pochette, et que ladite encoche est située à une extrémité de ladite ligne de pré-découpe.

Selon un autre mode de réalisation, ladite zone d'affaiblissement comprend deux découpes situées aux extrémités de la ligne de pré-découpe.

Avantageusement, ledit support imprégné est fixé par collage ou soudure à la partie de la pochette formant moyen de préhension.

De préférence, le support est imprégné en sursaturation de produit de manière apte à permettre le transfert du produit par pression et/ou par capillarité depuis la partie dudit support contenu dans la partie de la pochette formant moyen de préhension vers la partie dudit support apparente après séparation de la première partie de ladite pochette.

De plus, l'emballage comprend au moins une autre pochette enfermant hermétiquement un élément, notamment un autre support imprégné, un bandage, une compresse, un sparadrap ou un pansement, et comprenant des moyens d'ouverture de ladite pochette de manière à laisser apparaître au moins en partie ledit élément.

Selon un mode de réalisation, les moyens d'ouverture se présentent sous la forme d'une zone d'affaiblissement des matériaux constituant ladite autre pochette de manière à faciliter la séparation de cette pochette en une première et une deuxième partie.

Préférentiellement, ladite autre pochette est constituée d'au moins deux parois fixées l'une à l'autre et que les moyens d'ouverture permettent de désolidariser au moins partiellement par pelage lesdites parois.

D'autres caractéristiques et avantages de l'invention ressortiront de la description détaillée qui va suivre des modes de réalisation non limitatifs de l'invention, en référence aux figures annexées dans lesquelles :
- la figure 1 représente l'emballage selon l'invention lors de son ouverture ;
- la figure 2 représente l'emballage selon l'invention après ouverture ;
- la figure 3 représente l'emballage selon l'invention avant ouverture ;
- la figure 4 représente l'emballage en vue partielle en coupe selon l'axe I-I' de la figure 3 ;
- la figure 5 représente une coupe longitudinale de l'emballage selon un mode de réalisation de l'invention ;
- la figure 6 représente une coupe longitudinale de l'emballage selon un autre mode de réalisation de l'invention ; et
- la figure 7 représente l'ouverture de l'emballage selon un autre mode de réalisation de l'invention.

La présente invention concerne un emballage 1 pour support 2 imprégné de produit, support hermétiquement enfermé dans au moins une pochette 3.

Un tel emballage 1 est destiné à un usage unique et trouve son application dans les domaines de la pharmacie, la cosmétique, la parfumerie ou analogue dans le cas d'un support 2 imprégné d'un produit destiné à une application cutanée. En particulier, dans le domaine de la pharmacie, le produit peut être un désinfectant, un hydratant, un cicatrisant ou autre, et le support 2 est alors destiné au soin d'une plaie ou d'une blessure.

L'emballage selon l'invention trouve aussi son application dans les emballages 1 enfermant un support 2 imprégné d'un produit susceptible de salir les mains de l'utilisateur ou qui ne doit pas entrer en contact avec sa peau, par exemple une teinture, un détergeant, un produit nettoyant ou corrosif.

Ledit support 2 peut se présenter sous la forme d'un tampon 4 constitué de fibres naturelles, synthétiques ou en une combinaison des deux et est enfermé hermétiquement à l'intérieur de ladite pochette 3 de manière à préserver l'étanchéité des l'emballage 1.

Il convient de noter que l'étanchéité de la pochette 3 est primordiale, en particulier pour la protection et la conservation du produit dont le support 2 est imbibé. De plus, elle offre une barrière hermétique requise pour certains produits comme un cicatrisant, un hydratant ou un désinfectant.

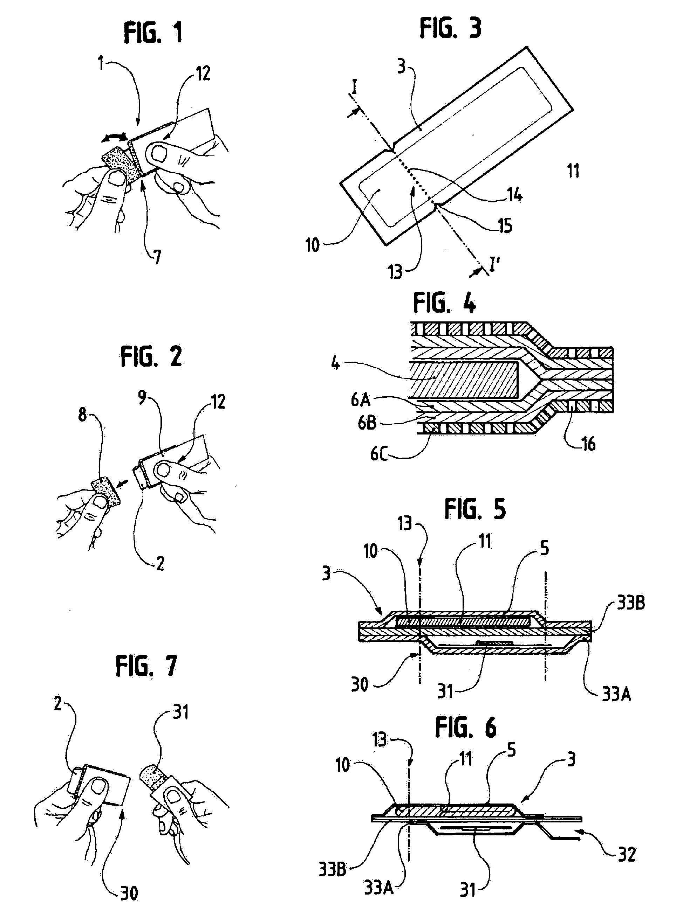

Cette protection est assurée au travers des différentes couches formant les parois 5 de ladite pochette 3, particulièrement visibles sur la figure 4. En particulier, les parois 5 peuvent être avantageusement constituée d'une superposition de couches 6A,6B,6C, comme un laminé ou un complexe de laminés de divers polymères et/ou aluminium, papier ou autre, par exemple un complexe laminé de polyester / aluminium / polyéthylène. Dans ce cas, des couches de polyester 6A et de polyéthylène 6C servent en outre à la protection de l'emballage 1 tandis que la couche d'aluminium 6B améliore la conservation du produit, notamment en le protégeant de la lumière et des gaz.

L'étanchéité de la pochette 3 est assurée au travers de la fixation de ses parois 5 au niveau de la périphérie de la pochette 3. Cette fixation peut être réalisée par soudure, collage ou thermocellage.

L'emballage 1 selon l'invention se caractérise entre autres par le fait que ladite pochette 1 comprend des moyens de séparation 7 d'au moins une première partie 8 par rapport à une deuxième partie 9 de la pochette 3. Lors de cette séparation, la première partie 8 laisse apparaître au moins partiellement ledit support 2 imprégné. En effet, la première partie 8 se détache de la deuxième partie 9 à la manière d'un capuchon, visible sur la figure 2, libérant une partie 10 dudit support 2 tandis que l'autre partie 11 du support 2 reste à l'intérieur de la deuxième partie 9 de la pochette 3.

Pour permettre au support de rester dans la deuxième partie 9, ce dernier se trouve être solidaire de celle-ci, en particulier en fixant ledit support 3 par collage, soudure ou analogue, à au moins une des faces intérieures des parois 5 correspondant à ladite deuxième partie 9 de la pochette 3. De cette manière, ledit support 2 est maintenu dans la partie 9 de la pochette 3 lors de l'ouverture de cette dernière.

De plus, cette deuxième partie 9 est apte à servir de moyen de préhension 12 dudit support 2 imprégné. Ainsi, la fixation dudit support 2 à cette partie 9 de la pochette 3 empêche le support d'en sortir lors de sa manipulation. Ces moyens de préhension 12 permettent aussi à l'utilisateur de manipuler l'emballage 1, et ainsi le support 2, sans le toucher directement, évitant tout risque de souillure du support au contact des mains ou de se salir ces dernières.

Lesdits moyens de séparation 7 se présentent sous la forme d'au moins une zone d'affaiblissement 13 des matériaux constituant ladite pochette 3 de manière à faciliter la séparation de la première 8 et de la deuxième partie 9 de la pochette 3.

Selon un mode de réalisations de l'emballage 1 selon l'invention, la zone d'affaiblissement 13 comprend au moins une ligne de pré-découpe 14 s'étendant au moins en partie transversalement à ladite pochette 3. Cette ligne de pré-découpe 14 comprend des découpes ou des perforations 16 au moins partielle desdits matériaux constituant ladite pochette 2 de manière à conserver son étanchéité. Plus particulièrement, les découpes ou perforations 16 sont réalisées au travers des couches supérieure et/ou inférieure des parois 5 de la pochette 3, notamment au travers de la couche extérieure et/ou intérieure sans altérer la couche centrale. La couche centrale reste alors intacte et assure l'étanchéité de la pochette 2.

Les deux couches supérieures peuvent être, perforées et la paroi intérieure reste intacte, seule la couche supérieure et la couche intérieure peuvent être perforées, soit les deux en conservant intact la couche centrale.

Selon un autre mode de réalisation, la zone d'affaiblissement 13 comprend au moins une encoche 15 située en périphérie de ladite pochette 3. Cette encoche 15 consiste à sectionner une ou plusieurs couches 6A,6B,6C sur tout ou partie de leur épaisseur de manière à former une fente facilitant la découpe des partie 8 et 9 de la pochette 3. En particulier, la zone d'affaiblissement 1 peut comprendre deux encoches 15 situées sur des bords opposés de la pochette 3.

Selon encore un autre mode, de réalisation, visible sur la figure 3, la zone d'affaiblissement 13 comprend une ligne de pré-découpe 14 combiné à au moins une encoche 15 située en périphérie de ladite pochette 3, en particulier deux encoches 15 situées à chaque extrémité de la ligne de pré-découpe 14.

La ligne de pré-découpe 14 et/ou les encoches 15 peuvent être réalisées sur les parois 5 de la pochette 3 avant le façonnage de cette dernière.

Il convient de noter que la pochette 3 est remplie d'une quantité de produit suffisante pour être extraite de la pochette 3 par pression sur ladite partie 9 formant moyen de préhension 12 et/ou par capillarité le long dudit support 2 imprégné. Ainsi, lors de l'utilisation du support imprégné 2, il est possible à l'utilisateur d'augmenter selon ses besoins la quantité de produit sur la partie apparente 10 du support 2.

Un autre avantage de l'emballage 1 selon l'invention réside dans le fait qu'il comprend au moins une autre pochette 30 enfermant hermétiquement un élément 31, notamment un autre support imprégné, un bandage, une compresse, un sparadrap ou un pansement ou analogue. L'emballage 1 selon l'invention pourvu de cette autre pochette 30 est particulièrement visible sur les figures 5, 6 et 7. De manière générale, cet élément 31 est destiné à une utilisation complémentaire à l'utilisation du support imprégné 2 contenu dans la pochette 3. Par exemple, l'autre pochette 30 peut contenir une lingette lustrante alors que la pochette 3 contient un tampon imprégné de cirage. Dans un autre cas, la pochette 3 contient un détergeant alors que l'autre pochette 30 enferme une lingette absorbante.

Cette pochette 30 est réalisée de manière similaire à la pochette 3, par une succession de couches tel un laminé ou un complexe laminé. Selon le type d'élément 31 enfermé dans ladite pochette 30, cette dernière pourra être prévue hermétique et/ou étanche. Elle comprend en outre des moyens d'ouverture 32 de ladite pochette de manière à laisser apparaître au moins en partie ledit élément 31.

Il convient de noter que, suivant les modes de réalisation, les deux pochettes 3 et 30 peuvent être juxtaposées l'une à l'autre ou superposées. Dans ce dernier cas, les pochettes 3 et 30 peuvent être solidaire au travers d'une unique paroi commune ou au travers d'une fixation des deux parois de chaque pochette qui sont en contact.

Selon un mode réalisation visible sur la figure 5, les moyens d'ouverture 32 de la pochette 30 se présentent sous la forme d'une zone d'affaiblissement des matériaux constitutifs de ladite pochette 30. Cette zone d'affaiblissement peut être réalisée de manière similaire à la zone d'affaiblissement 13 de la pochette 3. En particulier, cette zone d'affaiblissement permet la séparation de la pochette 30 en deux parties, laissant apparaître une partie dudit élément 31 en vue de son utilisation ou de son extraction de la pochette 30. L'utilisation est similaire à celle décrite dans le cas de la pochette 3, l'élément 31 étant fixé à l'intérieur de ladite pochette 30. Il est aussi possible à l'utilisateur d'extraire ledit élément 31 de la pochette 30, notamment dans le cas d'un sparadrap, d'un pansement ou analogue.

Selon un autre mode de réalisation visible sur la figure 6, la pochette 30 est constituée d'au moins deux parois 33A et 33B fixées l'une à l'autre et que les moyens d'ouverture 32 permettent de désolidariser au moins partiellement par pelage lesdites parois 33A et 33B. De cette manière, la pochette 30 peut être ouverte entièrement pour permettre, suivant le cas, la libération de l'élément 31 dans son entier ou l'utilisation de l'élément 31, notamment un support imprégné de produit tel un tampon, une compresse ou analogue, sur une plus grande surface. Dans ce dernier cas, l'élément 31 peut être replié et fixé sur chacune des parois 33A et 33B de la pochette 30 de sorte que l'ouverture de ces dernières déploie l'élément 31.

Selon les modes de réalisation, comme visible sur les figures 5 à 7, les pochettes 3 et 30 peuvent être assujetties l'une à l'autre de manière à mettre en opposition l'ouverture de chacune des pochettes 3 et 30. Ainsi, l'utilisateur a peu de chance de se tromper d'ouverture.

A l'inverse, dans le cas de zone d'affaiblissement 13 comprenant une ligne de pré-découpe 14 et/ou des encoches 15, les deux pochettes 3 et 30 peuvent prévoir une ouverture d'un même côté, soit au travers d'une zone d'affaiblissement 13 commune aux deux pochettes 3 et 30, soit au travers de plusieurs zone d'affaiblissement pour une ouverture successive de la première pochette 3 puis de l'autre pochette 30. Par exemple, et de manière non limitative, la première pochette 3 comprend une zone d'affaiblissement laissant apparaître ledit support imprégné 3 tandis que ce dernier est fixé à une partie de la pochette 3 située avant la zone d'affaiblissement de la seconde pochette 30. De cette façon, l'ouverture de la seconde pochette 30 extrait le support imprégné 2 de l'autre pochette 3.

Il convient de noter que l'emballage 1 peut comprendre une juxtaposition ou une superposition de plus de deux pochettes, ces dernières pouvant être de toute forme. En particulier, dans le cas de trois pochettes superposées, ces dernières peuvent se présenter sous une forme triangulaire de manière à positionner l'ouverture de chaque pochette sur un bord dudit triangle.

L'emballage 1 selon l'invention offre une facilité d'ouverture des pochettes le constituant tout en offrant une manipulation simple et propre du support 3 y enfermé.

## Revendications

1. Emballage (1) pour support (2) imprégné d'un produit, support (2) hermétiquement enfermé dans au moins une pochette (3), cette dernière comprenant des moyens de séparation (7) d'au moins une première partie (8) de la pochette (3) laissant apparaître au moins partiellement ledit support (2) imprégné solidaire d'une deuxième partie (9) de ladite pochette (3) apte à servir de moyen de préhension (12) dudit support (2) imprégné, lesdits moyens de séparation (7) se présentant sous la forme d'au moins une zone d'affaiblissement (13) des matériaux constituant ladite pochette (3) de manière à faciliter la séparation de la première (8) et de la deuxième (9) partie de ladite pochette (3) au travers d'au moins une ligne de pré-découpe (14) s'étendant au moins en partie transversalement à ladite pochette (3) et comprenant des découpes ou des perforations (16) au moins partielles desdits matériaux constituant ladite pochette (3) de manière à conserver l'étanchéité de ladite pochette (3), **caractérisé par le fait que** ladite pochette comprend des parois constituées d'une superposition de trois couches (6A,6B,6C) et que lesdites découpes ou perforations (16) sont réalisées au travers de deux desdites couches, tout en conservant une des couches intacte.

2. Emballage (1) selon la revendication 1, **caractérisé par le fait que** lesdites découpes ou perforations (16) sont réalisées au travers de la couche intérieure (6A).

3. Emballage (1) selon l'une quelconques des revendications 1 ou 2, **caractérisé par le fait que** lesdites découpes ou perforations (16) sont réalisées au travers de la couche supérieure (6C), laissant intact la couche centrale (6B).

4. Emballage selon la revendication 2, **caractérisé par le fait que** ladite zone d'affaiblissement (13) comprend au moins une encoche (15) située en périphérie de ladite pochette (3).

5. Emballage (1) selon l'une quelconque des revendications précédentes, **caractérisé par le fait que** le support (2) est imprégné en sursaturation de produit de manière apte à permettre le transfert du produit par pression et/ou par capillarité depuis la partie (10) dudit support (2) contenu dans la partie (9) de la pochette (3) formant moyen de préhension (12) vers la partie (10) dudit support (2) apparente après séparation de la première partie (8) de ladite pochette (3).

6. Emballage (1) selon l'une quelconque des revendications précédentes, **caractérisé par le fait qu'**il comprend au moins une autre pochette (30) enfermant hermétiquement un élément (31), notamment un autre support imprégné, un bandage, une compresse, un sparadrap ou un pansement, et. comprenant des moyens d'ouverture (32) de ladite pochette (30) de manière à laisser apparaître au moins en partie ledit élément (31).

7. Emballage (1) selon la revendication 4, **caractérisé par le fait que** les moyens d'ouverture (32) se présentent sous la forme d'une zone d'affaiblissement des matériaux constituant ladite autre pochette (30) de manière à faciliter la séparation de cette pochette (30) en une première et une deuxième partie.

8. Emballage (1) selon la revendication 5, **caractérisé par le fait que** ladite autre pochette (30) est constituée d'au moins deux parois (33A, 33B) fixées l'une à l'autre et que les moyens d'ouverture (32) permettent de désolidariser au moins partiellement par pelage lesdites parois (33A, 33B).

## Claims

1. Packaging (1) for a support (2) impregnated with a product, which support (2) is tightly enclosed in at least one envelope (3), the latter comprising means for separating (7) at least a first portion (8) of the envelope (3) exposing at least partially said impregnated support (2) integral with a second portion (9) of said envelope (3) capable of serving as means for seizing (12) said impregnated support (2), said separating means (7) being in the form of at least one weakening area (13) of the materials forming said envelope (3) so as to facilitate the separation of the first (8) and the second (9) portion of said envelope (3) through at least one pre-cut line (14) extending at least partially transversally to said envelope (3) and comprising at least partial cut-outs or perforations (16) of said materials forming said envelope (3) so as to preserve the tightness of said envelope (3), wherein said envelope comprises walls formed by a superposition of three layers (6A, 6B, 6C) and wherein said cut-outs or perforations (16) are made through two of said layers while keeping intact one of the layers.

2. Packaging (1) according to claim 1, wherein said cut-outs or perforations (16) are made through the inner layer (6A).

3. Packaging (1) according to any of claims 1 or 2, wherein said cut-outs or perforations (16) are made through the upper layer (6C), keeping intact the central layer (6B).

4. Packaging (1) according to claim 2, wherein said weakening area (13) comprises at least one notch (15) located at the periphery of said envelope (3).

5. Packaging (1) according to any of the preceding claims, wherein the support (2) is impregnated to supersaturation with product so as to be capable of permitting the transfer of product by compression and/or by capillarity from the portion (10) of said support (2) contained in the portion (9) of the envelope (3) forming the seizing means (12) to the portion (10) of said support (2) that is exposed after separation of the first portion (8) of said envelope (3).

6. Packaging (1) according to any of the preceding claims, which comprises at least another envelope (30) enclosing in a tight way an element (31), namely another impregnated support, a bandage, a dressing pad, a plaster or a dressing, and comprising means for opening (32) said envelope (30) so as to expose said element (31) at least partially.

7. Packaging (1) according to claim 4, wherein the opening means (32) are in the form of a weakening area of the materials forming said other envelope (30) so as to facilitate the separation of this envelope (30) into a first and a second portion.

8. Packaging (1) according to claim 5, wherein said other envelope (30) is comprised of at least two walls (33A, 33B) secured to each other and wherein the opening means (32) permit to separate said walls (33A, 33B) at least partially by peeling-off.

## Patentansprüche

1. Verpackung (1) für einen mit einem Produkt imprägnierten Träger (2), welcher Träger (2) in wenigstens einer Hülle (3) dicht eingeschlossen ist, wobei diese letztere Mittel zum Trennen (7) von wenigstens einem ersten Teil (8) der Hülle (3) umfasst, wodurch der besagte imprägnierte Träger (2), der fest mit einem zweiten Teil (9) der besagten Hülle (3) verbunden ist, der geeignet ist, als Mittel zum Greifen (12) des besagten imprägnierten Trägers (2) zu dienen, wenigstens zum Teil freigelegt wird, wobei die besagten Trennungsmittel (7) als wenigstens ein Schwächungsbereich (13) der der Hülle (3) bildenden Materialien ausgestaltet sind, sodass die Trennung des ersten (8) und des zweiten (9) Teils der besagten Hülle (3) durch wenigstens eine Vorschneidelinie (14) erleichtert wird, die sich wenigstens zum Teil quer zur besagten Hülle (3) erstreckt und wenigstens Teilausschnitte oder Teildurchbohrungen (16) der besagten, die besagte Hülle (3) bildenden Materialien umfasst, sodass die Dichtheit der besagten Hülle (3) beibehalten wird, **dadurch gekennzeichnet, dass** die besagte Hülle durch eine Überlagerung von drei Schichten (6A, 6B, 6C) gebildete Wände umfasst, und dass die besagten Ausschnitte oder Durchbohrungen (16) durch zwei der besagten Schichten hindurch gemacht sind, während eine der Schichten intakt gehalten wird.

2. Verpackung (1) nach Anspruch 1, **dadurch gekennzeichnet, dass** die besagten Ausschnitte oder Durchbohrungen (16) durch die innere Schicht (6A) hindurch gemacht sind.

3. Verpackung (1) nach irgendeinem der Ansprüche 1 oder 2, **dadurch gekennzeichnet, dass** die besagten Ausschnitte oder Durchbohrungen (16) durch die obere Schicht (6C) hindurch gemacht sind, während die mittlere Schicht (6B) intakt gehalten wird.

4. Verpackung (1) nach Anspruch 2, **dadurch gekennzeichnet, dass** der besagte Schwächungsbereich (13) wenigstens eine Einkerbung (15) umfasst, die sich am Umkreis der besagten Hülle (3) befindet.

5. Verpackung (1) nach irgendeinem der vorgehenden Ansprüche, **dadurch gekennzeichnet, dass** der Träger (2) bis zu Übersättigung mit Produkt imprägniert ist, so dass er geeignet ist, die Übertragung von Produkt durch Druck und/oder Kapillarität vom Teil (10) des besagten Trägers (2), der im die Greifmittel (12) bildenden Teil (9) der Hülle (3) enthalten ist, zum Teil (10) des besagten Trägers (2), der nach der Trennung des ersten Teils (8) der besagten Hülle (3) freigelegt ist, zu erlauben.

6. Verpackung (1) nach irgendeinem der vorgehenden Ansprüche, **dadurch gekennzeichnet, dass** sie eine weitere Hülle (30) umfasst, die ein Element (31), nämlich einen weiteren imprägnierten Träger, einen Bindeverband, eine Kompresse, einen Pflaster oder einen Verband, dicht einschliesst und Mittel zum Öffnen (32) der besagten Hülle (30) umfasst, sodass das besagte Element (31) wenigstens zum Teil freigelegt wird.

7. Verpackung (1) nach Anspruch 4, **dadurch gekennzeichnet, dass** die Öffnungsmittel (32) als ein Schwächungsbereich der die besagte weitere Hülle (30) bildenden Materialien ausgestaltet sind, sodass die Trennung dieser Hülle (30) in einen ersten und einen zweiten Teil erleichtert wird.

8. Verpackung (1) nach Anspruch 5, **dadurch gekennzeichnet, dass** die besagte weitere Hülle (30) aus wenigstens zwei Wänden (33A, 33B) besteht, die an einander befestigt sind, und dass die Öffnungsmittel (32) es erlauben, die besagten (33A, 33B) Wände durch Abziehen wenigstens zum Teil von einander zu trennen.
